(19) Europäisches Patentamt / European Patent Office / Office européen des brevets

(11) **EP 3 858 307 B1**

(12) **EUROPÄISCHE PATENTSCHRIFT**

(45) Veröffentlichungstag und Bekanntmachung des Hinweises auf die Patenterteilung:
**20.12.2023 Patentblatt 2023/51**

(21) Anmeldenummer: **21153935.8**

(22) Anmeldetag: **28.01.2021**

(51) Internationale Patentklassifikation (IPC):
**A61F 13/08** *(2006.01)*

(52) Gemeinsame Patentklassifikation (CPC):
**A61F 13/08; A61F 13/085**

(54) **KOMPRESSIONSSYSTEM ZUR AUSÜBUNG EINER EINSTELLBAREN KOMPRESSION AUF EINE KÖRPEREXTREMITÄT**

COMPRESSION SYSTEM FOR APPLYING ADJUSTABLE COMPRESSION TO A BODY EXTREMITY

SYSTÈME DE COMPRESSION DESTINÉ À L'EXERCICE D'UNE COMPRESSION RÉGLABLE SUR UNE EXTRÉMITÉ CORPORELLE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **03.02.2020 DE 102020102593**

(43) Veröffentlichungstag der Anmeldung:
**04.08.2021 Patentblatt 2021/31**

(73) Patentinhaber: **Julius Zorn GmbH**
**86551 Aichach (DE)**

(72) Erfinder: **Kammal, Achim**
**95497 Goldkronach (DE)**

(74) Vertreter: **Charrier Rapp & Liebau**
**Patentanwälte PartG mbB**
**Fuggerstraße 20**
**86150 Augsburg (DE)**

(56) Entgegenhaltungen:
| | |
|---|---|
| EP-A1- 1 803 425 | WO-A1-2014/066714 |
| WO-A1-2014/160572 | DE-A1-102009 016 513 |
| DE-U1-202015 107 145 | US-A1- 2005 192 524 |
| US-B1- 6 338 723 | |

**Beschreibung**

Die Erfindung betrifft ein Kompressionssystem zur Ausübung einer einstellbaren Kompression auf eine Körperextremität nach dem Oberbegriff des Anspruchs 1.

Aus dem Stand der Technik sind Kompressionsbandagen zum Anlegen an eine Körperextremität eines Patienten, beispielsweise an einem Arm oder einem Bein, bekannt, mit denen ein bestimmter Kompressionsdruck auf die Körperextremität ausgeübt werden kann. Durch Ausübung eines Kompressionsdrucks auf Körperextremitäten eines Patienten können chronisch venöse Insuffizienzen und Gefäßerkrankungen, wie z.B. venöse Abflussstörungen, Veneninsuffizienzen, Lymph-Ödeme, ulcus cruris venosum, etc. therapeutisch behandelt werden, um bspw. den venösen Blutfluss oder den Abfluss von gestauten Lymphflüssigkeiten zu fördern, wie von E. Rabe et.al. in der Leitlinie "Medizinische Kompressionstherapie der Extremitäten mit Medizinischem Kompressionsstrumpf (MKS), Phlebologischem Kompressionsverband (PKV) und Medizinischen adaptiven Kompressionssystemen (MAK)" der Deutschen Gesellschaft für Phlebologie e.V. (DGP), AWMF-Registernummer: 037-005 (verfügbar unter www.awmf.org/leitlinien/detail/ll/037-005.html) definiert.

Für die Ausübung eines vorgegebenen Kompressionsdrucks oder eines vorgegebenen Druckprofils mit einem von distal nach proximal abnehmenden Kompressionsdruck entlang einer Körperextremität können beispielsweise schlauchförmige Kompressionsartikel, wie z.B. Kompressionsstrümpfe oder -ärmel, eingesetzt werden. Der von diesen schlauchförmigen Kompressionsartikeln auf die Körperextremität ausgeübte Kompressionsdruck ist zum einen von der Elastizität des schlauchförmigen Kompressionsartikels in Umfangsrichtung und andererseits vom Umfang der Körperextremität abhängig. Bei vorgegebenem Umfang der Körperextremität kann mit einem solchen schlauchförmigen Kompressionsartikel immer nur ein bestimmter, vorgegebener Kompressionsdruck oder ein vorgegebenes Kompressionsprofil auf die Körperextremität ausgeübt werden.

Weiterhin sind aus dem Stand der Technik Kompressionsbandagen bekannt, die über mindestens ein Band oder eine Mehrzahl von Bändern verfügen, welche unter Dehnung der Bänder und/oder eines Grundkörpers, an dem die Bänder befestigt sind, um eine Körperextremität geschlungen und dort mittels Befestigungsmittel, beispielsweise Klettverschlüsse, festgelegt werden können. Der von solchen Kompressionsbandagen auf die Körperextremität ausgeübte Kompressionsdruck hängt dabei neben dem Umfang der Körperextremität von der Dehnung der Bänder bzw. des Grundkörpers der Kompressionsbandage ab, mit der diese an der Körperextremität festgelegt worden sind. Solche Kompressionsbandagen ermöglichen deshalb die Einstellung unterschiedlicher Kompressionsdrücke bzw. unterschiedlicher Kompressionsprofile entlang der Längsrichtung der Körperextremität, da der an bestimmten Stellen auf die Körperextremität ausgeübte Kompressionsdruck durch die Zugkraft, mit der die Dehnung der Bänder bzw. des Grundkörpers der Bandage herbeigeführt wird, entsprechend einem gewünschten Kompressionsdruck bzw. dem gewünschten Druckprofil ausgewählt werden kann.

Nachteilig bei derartigen Kompressionsbandagen ist allerdings, dass nach dem Anlegen der Bandage an eine Körperextremität der tatsächlich ausgeübte Kompressionsdruck nicht genau definiert ist und nicht mehr festgestellt werden kann.

Um hier Abhilfe zu schaffen, werden im Stand der Technik bereits Kompressionsbandagen vorgeschlagen, welche den auf die Körperextremität ausgeübten Kompressionsdruck anzeigen, beispielsweise mittels Markierungen, die über die gesamte Länge der Bandage auf deren Außenseite angebracht sind und aufgrund einer Änderung der Form oder des Abstands der Markierungen einen Rückschluss auf die Dehnung der Bänder bzw. des Grundkörpers der Bandage und damit bei bekanntem Umfang der Körperextremität auf den ausgeübten Kompressionsdruck ermöglichen.

So sind beispielsweise aus der US 6338723 B1 therapeutische Bekleidungsstücke zur Erzeugung einer Kompression auf eine Körperextremität bekannt, welche ein dehnbares Band oder eine Mehrzahl von dehnbaren Bändern enthalten, welche entlang einer elastischen Achse gedehnt und um die Körperextremität geschlungen und dort unter Dehnung festgelegt werden können, wobei auf einer Außenseite des Bands oder der Bänder Markierungen angebracht sind, die über einen Vergleich der Position der Markierungen mit einer Referenzposition eine Erfassung der Dehnung des elastischen Materials des Bands ermöglichen. Zur Erfassung der Dehnung wird mittels einer Messkarte mit einer Skala der Abstand der Markierungen von der Referenzmarkierung gemessen und aus der so erfassten Dehnung wird der ausgeübte Kompressionsdruck in Abhängigkeit des vorher gemessenen Umfangs der Körperextremität bestimmt. In einer Ausführungsform enthält das therapeutische Bekleidungsstück eine Mehrzahl von flexiblen Bändern, die miteinander über einen Grundkörper verbunden sind und unter Dehnung zur Ausübung eines Kompressionsdrucks an einer Körperextremität festgelegt werden können, wobei auf der Außenseite der Bänder Markierungen angebracht sind, die in der oben beschriebenen Weise in Verbindung mit einer Messkarte eine Erfassung der Dehnung der Bänder und daraus einen Rückschluss auf den Kompressionsdruck ermöglichen, der von den unter Dehnung an der Körperextremität angelegten Bändern ausgeübt wird. Dabei wird davon ausgegangen, dass der ausgeübte Kompressionsdruck umgekehrt proportional zum Umfang der Körperextremität ist und dass bei bekanntem Umfang mit der Messkarte die Dehnung gemessen werden kann, um eine der Dehnung entsprechende Kompression zu erzeugen.

Mit den aus der US 6338723 B1 bekannten therapeutischen Bekleidungsstücken ist es zwar möglich, den vom Bekleidungsstück auf die Körperextremität ausgeübten Kompressionsdruck zumindest innerhalb gewisser Fehlertoleranzen zu erfassen und die Anlegung des Bekleidungsstücks an der Körperextremität anzupassen, wenn der gewünschte Kompressionsdruck oder ein gewünschtes Kompressionsprofil entlang der Längsrichtung der Körperextremität nicht erhalten worden ist. Ein genaues und zielgerichtetes Anlegen des kompressiven Bekleidungsstücks an eine Körperextremität unter möglichst genauer Einhaltung eines vorgegebenen Kompressionsdrucks oder eines bestimmten Druckprofils wird durch dieses bekannte Kleidungsstück jedoch nicht ermöglicht. Insbesondere können keine definierten und reproduzierbaren Druckprofile des Kompressionsdrucks eingestellt werden, weil bspw. die Form und der Umfangsverlauf der Körperextremität nicht ausreichend berücksichtigt wird.

Aus der DE 10 2009 016 513 A1 ist eine segmental zirkulär adaptive Kompressionsmanschette bekannt, welche einen längsgeschlitzten elastischen Strumpf und eine Mehrzahl von handbetätigbaren Verschlüssen in Form von Klettbändern umfasst, die entlang von Längsschlitzrändern des Strumpfs alternierend zueinander angeordnet sind. Dabei ist der Kompressionsdruck an mindestens einem Segment der Kompressionsmanschette mittels eines Zugkraftmessgeräts und/oder eines Drucksensors messbar.

Hiervon ausgehend liegt der Erfindung daher die Aufgabe zu Grunde, ein elastisches Kompressionstextil aufzuzeigen, welches zielgerichtet unter Einhaltung eines vorgegebenen Kompressionsdrucks oder eines vorgegebenen Kompressionsprofils entlang der Längsrichtung der Körperextremität an einer Körperextremität angelegt werden kann, wobei der Kompressionsdruck bzw. das Kompressionsdruckprofil möglichst genau einstellbar sein soll. Weiterhin soll das elastische Kompressionstextil auf möglichst einfache und schnelle Weise unter Einhaltung des vorgegebenen Kompressionsdrucks bzw. des vorgegebenen Druckprofils an eine Körperextremität angelegt werden können. Das Anlegen des Kompressionstextils soll dabei insbesondere auch vom Patienten selbst durchführbar sein.

Diese Aufgaben werden mit einem Kompressionssystem mit den Merkmalen des Anspruchs 1 gelöst. Bevorzugte Ausführungsformen des Kompressionssystems sind in den abhängigen Ansprüchen aufgezeigt.

Das erfindungsgemäße Kompressionssystem umfasst ein zumindest bereichsweise elastisches Kompressionstextil mit einem Grundkörper und mindestens einem an dem Grundkörper angeordneten Band, welches über ein freies Ende verfügt, an dem erste Befestigungsmittel angeordnet sind, wobei das Kompressionstextil unter Dehnung um die Körperextremität schlingbar und unter Beibehaltung der Dehnung mittels der ersten Befestigungsmittel an der Körperextremität festlegbar ist und sich das Kompressionstextil beim Anlegen an die Körperextremität in Abhängigkeit einer zur Erzeugung der Dehnung auf das Band ausgeübten Zugkraft dehnt, so dass bei angelegtem Kompressionstextil eine von der Dehnung abhängige Kompression auf die Körperextremität ausgeübt wird. Zur Aufnahme der beim Anlegen an die Körperextremität auf das Band oder jedes Band ausgeübten Zugkraft umfasst das erfindungsgemäße Kompressionssystem eine Kraftmesseinrichtung, die mittels weiterer Befestigungsmittel abnehmbar an den freien Enden des Bands oder der Bänder befestigbar ist.

Zum Anlegen des zumindest bereichsweise elastischen Kompressionstextils an die Körperextremität wird in dem erfindungsgemäßen Kompressionssystem eine insbesondere manuell erzeugte Zugkraft über die Kraftmesseinrichtung zunächst auf ein Band des Kompressionstextils und anschließend ggf. nacheinander auf die weiteren in dem Kompressionstextil enthaltenen Bänder übertragen, wodurch sich das jeweilige Band und/oder der Grundkörper um eine von der aufgebrachten Zugkraft abhängige Strecke dehnt. Die über die Kraftmesseinrichtung auf das jeweilige Band ausgeübte Zugkraft wird dabei gleichzeitig von der Kraftmesseinrichtung erfasst und zweckmäßig über eine in der Kraftmesseinrichtung integrierte oder eine mit der Kraftmesseinrichtung verbundene Anzeige angezeigt. Sobald eine bestimmte, vorgegebene Zugkraft erreicht ist, welche dem vorgegebenen Kompressionsdruck entspricht, der am Ort des jeweiligen Bands auf die Körperextremität aufgebracht werden soll, wird das jeweilige Band unter Beibehaltung der durch die Zugkraft erzeugten Dehnung mittels der (ersten) Befestigungsmittel an der Körperextremität festgelegt. Dieser Vorgang wird zum vollständigen Anlegen des Kompressionstextils an einer Körperextremität für jedes Band in entsprechender Weise durchgeführt, bis alle Bänder des Kompressionstextils an der Körperextremität festgelegt und das Kompressionstextil dadurch an die Körperextremität angelegt ist.

Dabei ist es vorteilhaft, wenn zunächst das Band am distalen Ende des Kompressionstextils und danach die dazu benachbarten Bänder in proximaler Richtung entlang der Längsrichtung der Körperextremität angelegt werden. Dadurch kann insbesondere ein Kompressionsdruckprofil mit einem von distal nach proximal abnehmenden Kompressionsdruck eingestellt werden. Die hierfür notwendigen Zugkräfte, die beim Anlegen des Kompressionstextils auf die einzelnen Bänder auszuüben sind, können bei Kenntnis der Umfänge der Körperextremität an den jeweiligen Stellen der Bänder, die mittels eines Maßbands auf einfache Weise erfasst werden können, ermittelt werden. Somit kann mit einem vorgegebenen Kompressionstextil (mit bekannten Materialparametern und vorgegebener Geometrie, insbesondere bei bekannter Breite des oder jedes Bands) bei Kenntnis des Umfangsverlaufs der Körperextremität und einem vorgegebenen Kompressionsdruck bzw. einem vorgegebenen Kompressionsdruckprofil für jedes Band auf einfache Weise die zur Erzeugung der gewünschten Kompression erforderliche Zugkraft berechnet und über die Kraftmesseinrichtung genau eingestellt und auf das jeweilige Band übertragen werden.

Bei einem Kompressionstextil mit einer Mehrzahl von Bändern kann auch ein (maximaler) distaler Solldruck und ein gewünschter Druckverlauf des Kompressionsdrucks in Längsrichtung der Körperextremität vorgegeben werden.

Dabei kann in Abhängigkeit des Verlaufs des Umfangs der Körperextremität entlang der Längsrichtung die Breite B der einzelnen Bänder so ausgewählt werden, dass sich bei Anwendung einer einheitlichen Zugkraft auf das jeweilige Band der vordefinierte Druckverlauf unmittelbar ergibt. Dabei kann insbesondere ein stetiger Verlauf des Kompressionsdrucks eingestellt werden, der vom (maximalen) distalen Solldruck am distalen Ende des Kompressionstextils in proximaler Richtung kontinuierlich auf einen vorgegebenen Anteil des distalen Solldrucks (d.h. bis auf einen proximalen Restdruck) abnimmt, wie dies bei medizinischen Kompressionsstrümpfen der Fall ist.

Eine möglichst einfache und kostengünstige Ausgestaltung des erfindungsgemäßen Kompressionssystems wird ermöglicht, wenn ein mechanischer Kraftaufnehmer, insbesondere ein Federkraftmesser und bevorzugt ein Federkörper-Kraftaufnehmer als Kraftmesseinrichtung verwendet wird. Ein solcher mechanischer Kraftaufnehmer ermöglicht dabei eine einfache und vom jeweiligen Benutzer gut wahrnehmbare Anzeige der beim Anlegen des Kompressionstextils auf die Bänder ausgeübten Zugkraft, beispielsweise mittels einer Skala, die anzeigt, wie weit der mechanische Kraftaufnehmer bei Ausübung der Zugkraft aus einer kraftlosen Ruhestellung bewegt wird. Die Beschriftung bzw. die Einheit der Skala kann dabei dem jeweiligen Anwendungsfall angepasst werden. So kann bspw. die Skala unmittelbar die mittels der Kraftmesseinrichtung auf das Band übertragene Zugkraft (in Newton) anzeigen. Es ist jedoch auch möglich, dass die Skala die durch die Zugkraft auf das Band ausgeübte Dehnung (in cm) oder den bei Festlegung des Bands an der Körperextremität erzeugten Kompressionsdruck (in mmHg oder einer anderen Druckeinheit) anzeigt.

Alternativ zu einem mechanischen Kraftaufnehmer können auch elektrische Kraftmesseinrichtungen, insbesondere resistive, induktive, kapazitive oder piezoelekrische Kraftaufnehmer eingesetzt werden. Diese Kraftmesseinrichtungen zeichnen sich durch eine besonders gute Genauigkeit und Reproduzierbarkeit aus und ermöglichen die Ausgabe der ausgeübten Zugkraft über eine elektronische Anzeige. Dies wiederum ermöglicht über eine Kopplung der elektronischen Anzeige mit einem Datenverarbeitungssystem eine Dokumentation der beim Anlegen der Körperextremität auf die Bänder ausgeübten Zugkräfte bzw. des dadurch auf die Körperextremität ausgeübten Kompressionsdrucks bzw. des Druckprofils.

Insbesondere zur Anlegung des Kompressionstextils an längere Körperextremitäten, wie z.B. einem Bein, ist es vorteilhaft, wenn das Kompressionstextil drei oder mehr Bänder umfasst. Wenn an einer längeren Körperextremität, wie z.B. einem Bein ein Druckprofil mit einem von distal nach proximal abnehmendem Kompressionsdruck angelegt werden soll, ist es vorteilhaft, wenn das Kompressionstextil wenigstens vier Bänder oder mehr umfasst. Bei kürzeren Körperextremitäten, wie z.B. einem Unterschenkel oder einem Unterarm, kann es jedoch auch ausreichen, wenn das Kompressionstextil nur ein Band oder zwei Bänder enthält. Die Bänder sind dabei zweckmäßig so ausgestaltet, dass sie sich in Bandlängsrichtung zumindest so weit erstrecken, dass sie den Umfang der Körperextremität an der Stelle, an der das jeweilige Band vorgesehen wird, vollständig umgreifen und dadurch vollumfänglich um die Körperextremität geschlungen werden können. Die um die Körperextremität geschlungenen Bänder erstrecken sich bei angelegtem Kompressionstextil damit in Umfangsrichtung der Körperextremität. In Längsrichtung der Körperextremität liegen die Bänder nebeneinander, wobei benachbarte Bänder zweckmäßig durch einen Schlitz im Grundkörper des Kompressionstextils voneinander getrennt sind. Um einen möglichst durchgehenden Verlauf des Kompressionsdrucks in Längsrichtung der Körperextremität sicherzustellen, sind die Schlitze im Grundkörper dabei möglichst schmal ausgebildet. Insbesondere ist die Breite der Schlitze dabei wesentlich kleiner als die Breite der jeweiligen Bänder.

Am freien Ende weist jedes Band erste Befestigungsmittel auf, die beispielsweise als Klettverschlüsse, insbesondere als Hakenteil eines Klettverschlusses, ausgebildet sein können. Alternativ zu Klettverschlüssen können auch Druckknöpfe eingesetzt werden.

Um einen gleichmäßigen Kompressionsdruck in Umfangsrichtung auf die Körperextremität ausüben zu können, ist bevorzugt sowohl der Grundkörper als auch die daran angeordneten Bänder elastisch. Die Bänder können jedoch auch inelastisch sein oder eine geringere Elastizität aufweisen als der Grundkörper. Bänder mit einer geringeren Elastizität ermöglichen die Ausübung einer höheren Zugkraft und damit eines höheren Kompressionsdrucks und kommen beispielsweise dann zum Einsatz, wenn ein Kompressionsdruck von mehr als 40mm Hg auf die Körperextremität ausgeübt werden soll.

Zur Befestigung der Kraftmesseinrichtung an den freien Enden der Bänder sind zweite Befestigungsmittel vorgesehen, durch die die Kraftmesseinrichtung abnehmbar am freien Ende des jeweiligen Bands befestigt werden kann. Bei den zweiten Befestigungsmitteln kann es sich ebenfalls um Klettverschlüsse, insbesondere um ein Hakenteil eines Klettverschlusses, oder um Druckknöpfe oder Adhäsionsverschlüsse handeln. Die lösbar ausgebildeten zweiten Befestigungsmittel ermöglichen ein einfaches Befestigen und Abnehmen der Kraftmesseinrichtung an einem Band. Insbesondere bei der Verwendung von Klettverschlüssen als zweite Befestigungsmittel kann die Kraftmesseinrichtung schnell und einfach an ein Band befestigt und nach dem Festlegen des Bands über die ersten Befestigungsmittel wieder von diesem Band abgenommen werden, um dann wiederum über die zweiten Befestigungsmittel am nächsten Band befestigt zu werden.

In einem bevorzugten Ausführungsbeispiel weist die Kraftmesseinrichtung ein Verbindungselement, insbesondere in Form einer Lasche, zur lösbaren Befestigung der Kraftmesseinrichtung am freien Ende eines Bands auf. Dabei wird die Zugkraft beim Anlegen des Kompressionstextils an die Körperextremität von der Kraftmesseinrichtung über das Verbindungselement auf das mit dem Verbindungselement verbundene Band übertragen.

Vorteilhaft weist die Kraftmesseinrichtung einen Handgriff auf. Dies ermöglicht ein sicheres Ergreifen und eine gute Führung der Kraftmesseinrichtung beim Dehnen der einzelnen Bänder und beim Erfassen der Dehnung, die beim Anlegen des Kompressionstextils an einer Körperextremität auf die einzelnen Bänder ausgeübt wird.

In einer besonders bevorzugten Ausführungsform ist die Kraftmesseinrichtung als mechanischer Kraftaufnehmer, insbesondere als Federkraftmesser, bspw. als Federkörper-Kraftaufnehmer, ausgebildet und umfasst einen Handgriff oder eine Schlaufe. Bei dieser Ausführungsform kann die Kraftmesseinrichtung von einer Person, insbesondere der Person, die das Kompressionstextils an eine eigene Körperextremität anlegt, oder von einer Hilfsperson, über den Handgriff oder die Schlaufe leicht und sicher ergriffen und während der Ausübung einer Zugkraft auf eines der Bänder des Kompressionstextils sicher geführt werden. Gleichzeitig kann während der Dehnung eines der Bänder die hierfür aufgewendete Zugkraft von der Kraftmesseinrichtung abgelesen werden, bspw. auf einer Skala des mechanischen Kraftaufnehmers. Die Kraftmesseinrichtung dient bei dieser Ausführungsform auch als Anlege-oder Anziehhilfe, die ein erleichtertes Anlegen des Kompressionstextils an eine Körperextremität des Patienten ermöglicht. Dies ist vor allem für ältere Patienten vorteilhaft, die selbst, d.h. ohne eine Hilfsperson, das Kompressionstextil an eine eigne Körperextremität, z.B. ein Bein, anlegen müssen. Ältere Patienten leiden häufig an Komorbiditäten, wie z.B. Arthrose der Hände oder Gicht, die mit starken Schmerzen verbunden sind, welche ein Ergreifen und Dehnen der dünnen Zugbänder mit den Fingern erschweren oder unmöglich machen. Der Griff oder die Schlaufe können dagegen sicher im Handinneren liegen und so eine gute Kraftübertragung ermöglichen. Dabei ist die Kraftmesseinrichtung bevorzugt über ein Verbindungselement, bspw. in Form einer Lasche oder einem anderen Befestigungsmittel, lösbar mit einem der Bänder verbindbar. Das Verbindungselement ist bspw. über eine lösbare Klettverbindung an den Bändern des Kompressionstextils befestigbar. Es ist jedoch auch möglich, direkt an der Kraftmesseinrichtung die zweiten Befestigungsmittel, bspw. einen Klettverschluss oder Druckknöpfe, anzuordnen, um die Kraftmesseinrichtung lösbar an den freien Enden der Bänder zu befestigen. Dadurch können die Bänder beim Anlegen des Kompressionstextils an eine Körperextremität, eins nach dem anderen, unter Verwendung der Kraftmesseinrichtung mit einer definierten Zugkraft, die an der Kraftmesseinrichtung abgelesen werden kann, gedehnt und unter Dehnung am Kompressionstextil befestigt werden. Dabei dient die Kraftmesseinrichtung mit dem Griff oder der Schlaufe als Anziehhilfe, die eine leichte und sichere Übertragung der ausgeübten Zugkraft auf das mit der Kraftmesseinrichtung über das Verbindungselement bzw. über das Befestigungsmittel verbundene Band ermöglicht. Diese und weitere Merkmale sowie Vorteile und technische Wirkungen des erfindungsgemäßen Kompressionssystems zur Ausübung einer einstellbaren Kompression auf eine Körperextremität ergeben sich aus dem nachfolgend unter Bezugnahme auf die begleitenden Zeichnungen näher beschriebenen Ausführungsbeispiel. Die Zeichnungen zeigen:

Fig. 1:    Darstellung eines (nicht angelegten) Kompressionstextils mit einem Grundkörper und einer Mehrzahl von daran angeordneten Bändern, wobei die Innenseite des Kompressionstextils gezeigt ist, die beim Anlegen an eine Körperextremität mit dieser in Berührung kommt;

Fig. 2:    Darstellung einer Kraftmesseinrichtung zur Aufnahme der beim Anlegen des Kompressionstextils von Fig. 1 an eine Körperextremität ausgeübten Zugkraft, wobei die Kraftmesseinrichtung in Figur 2a im Grundzustand und in Figur 2b in einem zugkraftbelasteten Zustand gezeigt ist;

Fig. 3:    Schematische Darstellung der einzelnen Schritte zum Anlegen des Kompressionstextils von Fig. 1 an einer Körperextremität eines Patienten unter Verwendung der Kraftmesseinrichtung von Fig. 2;

Fig. 4:    Darstellung der standardisierten Messpunkte des Kompressionsdrucks von medizinischen Kompressionsstrümpfen, gemäß RAL-GZ 387/1.

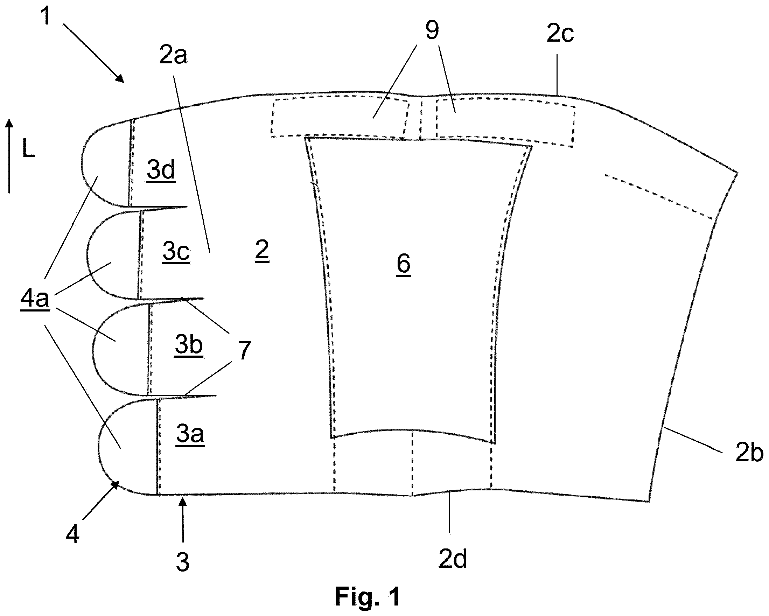

Das in Figur 1 in einer Draufsicht auf die Innenseite gezeigte Kompressionstextil umfasst einen Grundkörper 2 aus einem elastischen Material. Bei dem elastischen Material kann es sich bspw. um ein Textilgewebe aus Polyamid und Elasthan, insbesondere mit 87% Polyamid und 13% Elasthan, bezogen auf das Gewicht des Textilgewebes handeln. Das elastische Material weist dabei eine materialspezifische Dehnungscharakteristik auf, die zweckmäßig eine maximale Dehnung des Materials im Bereich von 15 bis 50 % der ungedehnten Länge ermöglicht. Bevorzugt ist die maximale Dehnung entweder zwischen 15 und 20 % oder zwischen 25 und 35%.

Der Grundkörper 2 weist eine in Figur 1 sichtbare Innenseite sowie eine Außenseite auf, wobei die Innenseite bei an einer Körperextremität angelegtem Kompressionstextil 1 der Körperextremität zugewandt ist. Der Grundkörper 2 umfasst dabei einen ersten Endabschnitt 2a und einen gegenüberliegenden zweiten Endabschnitt 2b sowie eine proximale Kante 2c und eine distale Kante 2d. Der zweite Endabschnitt 2b weist eine in Längsrichtung L des Grundkörpers 1 (von distal nach proximal) verlaufende glatte Kante auf, die zweckmäßig mit der distalen Kante 2d einen Winkel einschließt, der größer als 90° ist und insbesondere im Bereich von 100° bis 120° liegt. Am ersten Endabschnitt 2a sind mehrere Bänder 3a, 3b, 3c, 3d angeordnet. Die Bänder bestehen dabei zweckmäßig aus demselben elastischen Material wie der Grundkörper 2, können jedoch auch aus einem unelastischen Material oder aus einem weniger dehnbaren Material als der Grundkörper gefertigt sein. Zweckmäßig sind die Bänder 3a, 3b, 3c, 3d durch Schlitze 7 in dem ersten Endabschnitt 2a des Grundkörpers 2 ausgebildet und voneinander getrennt. Die Länge der Schlitze 7 und damit die Länge der Bänder ist dabei bevorzugt wesentlich kleiner als die Ausdehnung des Grundkörpers 2 in Querrichtung (Umfangsrichtung). Bevorzugt beträgt die Länge der Bänder 3a - 3d zwischen 2cm und 5 cm.

In dem hier zeichnerisch dargestellten Ausführungsbeispiel des Kompressionstextils 1 sind insgesamt vier Bänder 3a - 3d vorgesehen, welche in Längsrichtung L des Grundkörpers 2 nebeneinander liegend angeordnet sind, wobei benachbarte Bänder (3a, 3b; 3b, 3c; etc.) jeweils durch einen Schlitz 7 in dem Grundkörper 2 voneinander getrennt sind. Durch die Ausbildung der Bänder 3a - 3d durch die schmalen Schlitze 7 liegen benachbarte Bänder direkt nebeneinander, d.h. zwischen der Unterkante eines oberen (proximalen) Bands und der Oberkante des sich distal daran anschließenden Bands liegt lediglich ein geringer Zwischenraum, der durch einen Schlitz 7 gebildet und durch die Schlitzbreite bestimmt ist.

Jedes der Bänder 3a - 3d ist an einem ersten Ende mit dem Grundkörper 2 verbunden und weist ein dem ersten Ende gegenüberliegendes freies Ende auf. Am freien Ende verfügt jedes Band 3a - 3d über Befestigungsmittel 3, über welche das jeweilige Band an der Außenseite des Grundkörpers 2 festlegbar ist. Zweckmäßig sind die Befestigungsmittel 3 durch Klettverschlüsse 4a, 4, 4c, 4d an jedem freien Ende der Bänder 3a, 3b, 3c, 3d gebildet, wobei bevorzugt Hakenteile der Klettverschlüsse an den freien Enden der Bänder angeordnet sind und die Außenseite des Grundkörpers 2 mit Schlaufen zum Anhaften der Hakenteile der Klettverschlüsse ausgebildet ist. Die Befestigungsmittel 3 können jedoch auch durch andere Mittel, wie z. B. Druckknöpfe, gebildet sein.

An der in Figur 1 gezeigten Innenseite des Bandagenkörpers 2 ist eine Anziehhilfe in Form eines schlauchförmigen Einsatzes 6 angeordnet, wie aus Figur 1 ersichtlich. Der schlauchförmige Einsatz 6 ist bspw. durch einen elastischen, bevorzugt nahtlosen Textilschlauch gebildet, welcher an der Innenseite des Bandagenkörpers 2 befestigt ist, bspw. durch Vernähen. Das Textilmaterial des schlauchförmigen Einsatzes 6 ist dabei wesentlich dünner ausgebildet als das elastische Material des Bandagenkörpers 1 und besteht zweckmäßig ebenfalls aus einer Mischung von Polyamid und Elasthan.

An der Innenseite des Bandagenkörpers 2 ist am proximalen Randabschnitt nahe der proximalen Kante ein Haftband 9 angeordnet, welches sich in Querrichtung (Umfangsrichtung) des Bandagenkörpers 2 erstreckt.

Zum Anlegen des Kompressionstextils 2 an eine Körperextremität eines Patienten wird der Grundkörper 2 um die Körperextremität geschlungen und mittels der Bänder 3 (zunächst provisorisch) fixiert. Hierfür schlüpft der Patient mit der Körperextremität in den schlauchförmigen Einsatz 6 an der Innenseite des Grundkörpers 2. Danach wird der zweite Endabschnitt 2b des Grundkörpers 2 um eine Hälfte der Körperextremität geschlungen und dort (auf der Außenseite des schlauchförmigen Einsatzes 6 aufliegend) festgehalten. Anschließend wird der erste Endabschnitt 2a, an dem die Bänder 3 angeordnet sind, um die zweite Hälfte der Körperextremität geschlungen und die Bänder 3 werden mittels der ersten Befestigungsmittel 4 auf der Außenseite des Grundkörpers 2 zunächst provisorisch befestigt.

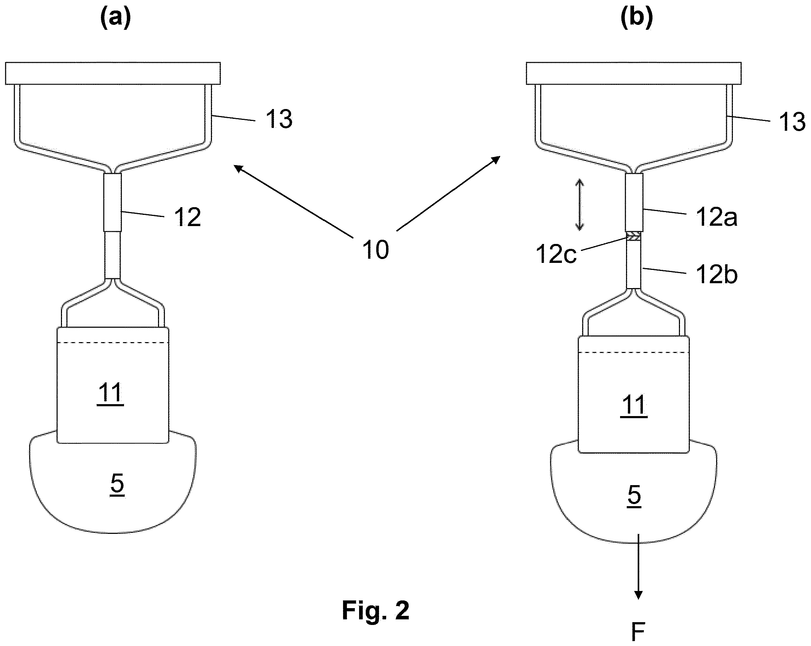

Um einen definierten Kompressionsdruck durch das angelegte Kompressionstextil 1 auf die Körperextremität auszuüben, wird die in Figur 2 gezeigte Kraftmesseinrichtung 10 verwendet.

Die Kraftmesseinrichtung 10 umfasst einen Handgriff 13, einen daran befestigten Kraftaufnehmer 12 und ein mit dem Kraftaufnehmer 12 verbundenes Verbindungselement 11, das in dem gezeigten Ausführungsbeispiel als Lasche ausgebildet ist. An dem Verbindungselement 11 sind abnehmbare zweite Befestigungsmittel 5 angeordnet. Bei dem zweiten Befestigungsmittel 5 kann es sich bspw. um ein Klettverschlusspad handeln, das auf einer Seite Hakenteile aufweist, welche an einer Fläche des Verbindungselements 11 lösbar befestigbar sind. Die Fläche des Verbindungselements 11 weist hierfür ein zu den Hakenteilen korrespondierendes Flauschteil auf. Die zweiten Befestigungsmittel 5 können auch fest an dem Verbindungselement 11 angeordnet, bspw. angenäht, oder in dieses integriert sein, bspw. als Flauschpad eines Klettverschlusses.

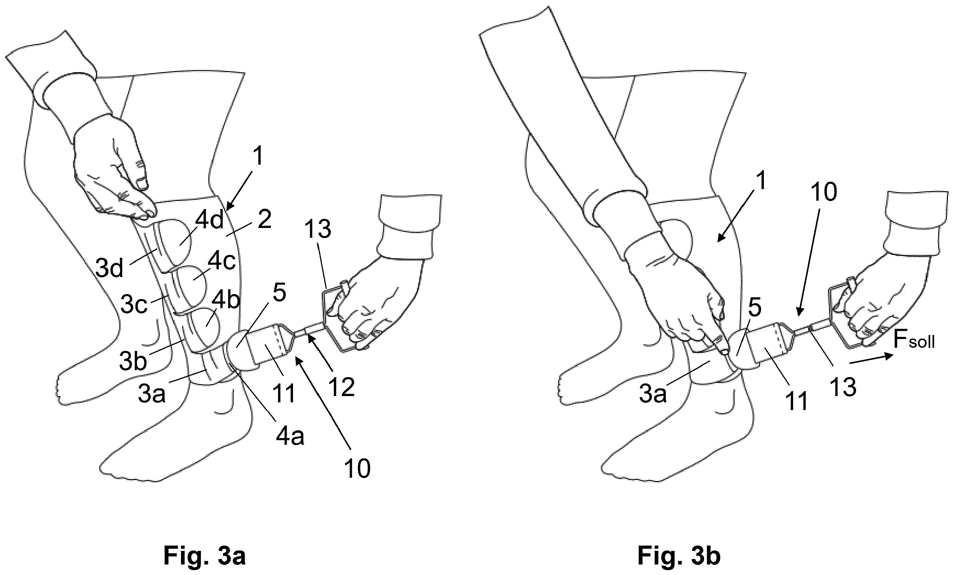

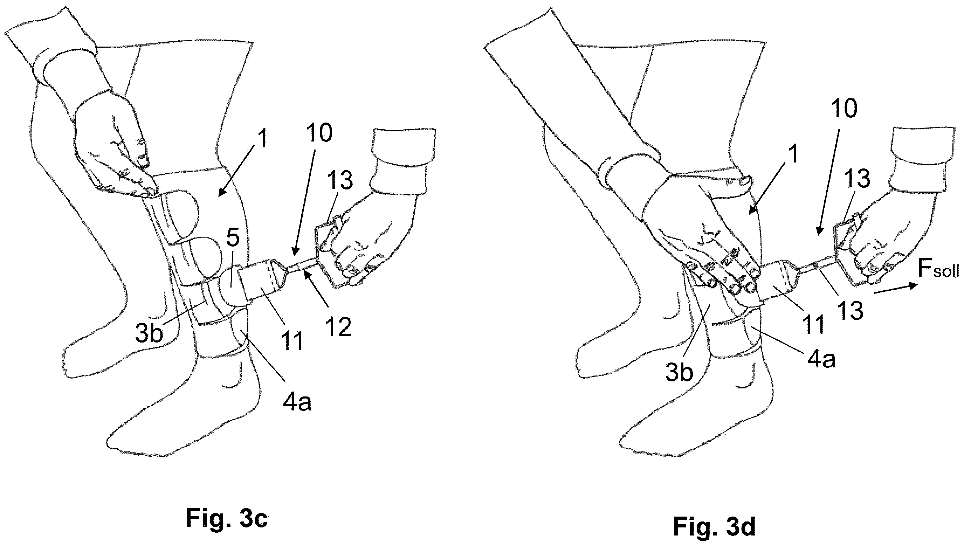

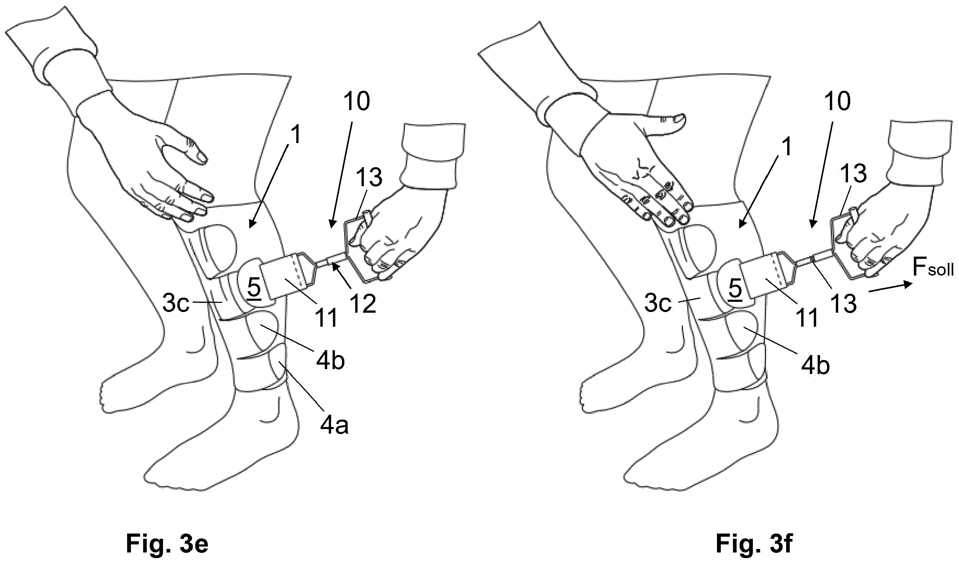

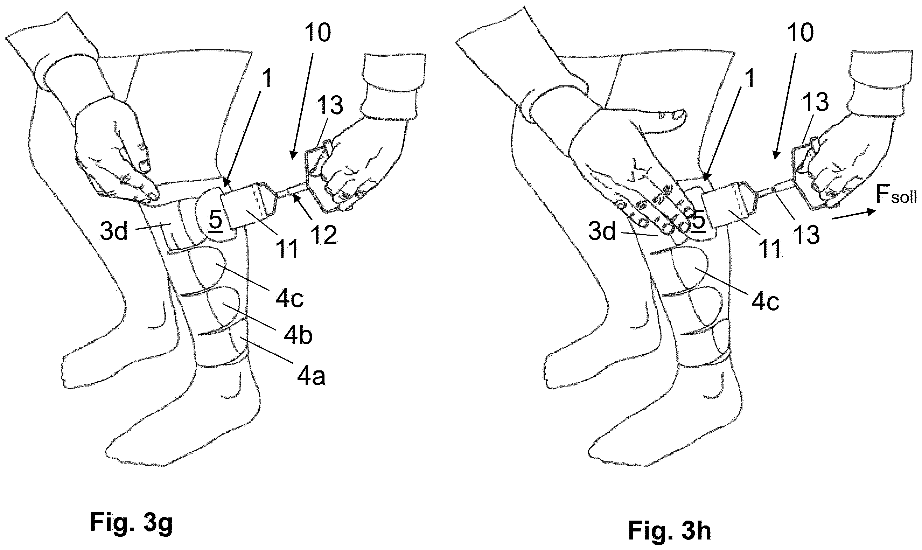

Der Kraftaufnehmer 12 ist in dem in Figur 2 gezeigten Ausführungsbeispiel als mechanischer Federkraftaufnehmer mit zwei ineinandergreifende Zylinder 12a, 12b ausgebildet, wobei der innere Zylinder 12b gegen die Rückstellkraft einer im äußeren Zylinder 12a angeordneten Feder aus dem äußeren Zylinder 12a herausziehbar ist. Die Strecke, um die der innere Zylinder 12b aus dem äußeren Zylinder 12a herausgezogen wird, stellt ein Maß für die zum Herausziehen aufgewendete Zugkraft F dar und wird über eine Skala 12c angezeigt, wie in Figur 2b gezeigt. In Figur 3 sind schematisch die Schritte zum Anlegen des Kompressionstextils 2 an eine Körperextremität eines Patienten zur Erzeugung eines vorgegebenen Kompressionsdrucks oder eines Druckprofils entlang der Längsrichtung L dargestellt, wobei es sich bei der Körperextremität beispielhaft um einen Unterschenkel K handelt.

Die Figuren 3a und 3b zeigen das Anlegen und Befestigen des distalen (unteren) Bands 3a des Kompressionstextils 1 von Figur 1 am Unterschenkel K unter einer vordefinierten Dehnung des zumindest teilweise elastischen Kompressionstextils 1. Hierzu wird zunächst die Kraftmesseinrichtung 10 mittels der zweiten Befestigungsmittel 5 auf der Außenseite des freien Endes des distalen Bands 3a befestigt, wie in Figur 3a gezeigt. Die ersten Befestigungsmittel 4a, mit denen das Band 3a zunächst provisorisch auf der Außenseite des Grundkörpers 2 befestigt worden ist, werden danach gelöst und es wird über den Handgriff 13 eine vordefinierte Zugkraft Fsoll manuell auf die am freien Ende des distalen Bands 3a befestigte Kraftmesseinrichtung 10 ausgeübt. Die Richtung der Zugkraft liegt dabei zweckmäßig in Umfangrichtung der Körperextremität (Unterschenkel U) oder weist zumindest eine Komponente in Umfangrichtung auf. Die manuell auf die Kraftmesseinrichtung 10 ausgeübte Zugkraft wird über das Verbindungselement 11 auf das damit verbundene Band 3a übertragen, so dass die Zugkraft Fsoll auf das Band 3a wirkt. Dadurch wird eine vordefinierte Dehnung auf das Kompressionstextil 1 ausgeübt. Der Betrag der ausgeübten Zugkraft F wird an der Skala 12c angezeigt, wie in Figur 3b gezeigt. Die durch die Zugkraft erzeugte vordefinierte Dehnung des Kompressionstextils 1 hängt von den Materialeigenschaften des Materials ab, aus dem das Band 3a und der Grundkörper 2 gefertigt sind.

Wenn die über die Kraftmesseinrichtung 10 auf das distale Band 3a ausgeübte Zugkraft dem Wert der vordefinierten Zugkraft Fsoll entspricht, wird das freie Ende des distalen Bands 3a wieder durch die ersten Befestigungsmittel 4a an der Außenseite des Grundkörpers 2 fixiert, indem bspw. die Hakenteile der ersten Befestigungsmittel 5 manuell mit einem Finger auf die flauschige Außenseite des Grundkörpers 2 gepresst werden, wie in Figur 2b gezeigt. Damit ist das Kompressionstextil 1 im distalen Abschnitt unter einer vordefinierten Dehnung an der Körperextremität (Unterschenkel U) fixiert und übt einen der Dehnung entsprechenden Kompressionsdruck aus, dessen Betrag vom Umfang der Körperextremität in diesem Abschnitt abhängig ist.

Danach werden die zweiten Befestigungsmittel 5 vom distalen Band 3a gelöst und der in den Figuren 3a und 3b gezeigte Vorgang wird in der oben beschriebenen Weise zunächst am darüber liegenden Band 3b und danach an den weiteren Bändern 3c und 3d, die sich in Längsrichtung L proximal anschließen, bis einschließlich des proximalen (oberen) Bands 3d wiederholt, wie in den Figuren 3c und 3d sowie in den Figuren 3e bis 3h dargestellt. Dadurch wird jedes Band 3a, 3b, 3c, 3d unter einer vordefinierten Dehnung an der Körperextremität fixiert und übt einen der Dehnung entsprechenden Kompressionsdruck aus, dessen Betrag jeweils von der Dehnung an der entsprechenden Position des jeweiligen Bands 3 und vom Umfang der Körperextremität in diesem Abschnitt abhängig ist. Dabei können die Dehnungen bzw. der dadurch hervorgerufene Kompressionsdruck im Bereich der einzelnen Bänder 3a, 3b, 3c, 3d jeweils gleich oder auch unterschiedlich sein. Insbesondere kann ein vordefinierter Druckverlauf mit einem in Längsrichtung L von distal nach proximal abnehmenden Kompressionsdruck eingestellt werden.

Die zur Erzeugung der vordefinierten Dehnung des Kompressionstextils 1 manuell aufzuwenden Zugkräfte können dabei vom Benutzer des Kompressionstextils 1 selbst (wie in Figur 3 gezeigt) oder auch von einer Hilfsperson aufgebracht werden.

Der bei einer bestimmten Zugkraft auf eines der Bänder 3 auf die Körperextremität ausgeübte Kompressionsdruck kann durch folgenden Zusammenhang beschrieben werden:

$$p = 2\pi\, F / (U \cdot B),$$

wobei p der Kompressionsdruck, F die Zugkraft in Umfangsrichtung, U der Umfang der Körperextremität an der Stelle des betreffenden Bands 3 und B die Breite des Bands (Ausdehnung in Längsrichtung L) ist.

Wenn das Band 3 bzw. das Kompressionstextil 1 über eine (sich in Umfangsrichtung erstreckende) (Band-)Länge 1 elastisch ist, wird das Band 3 bzw. das Kompressionstextil 1 durch die Zugkraft F um eine Dehnung s gedehnt:

$$s = F \cdot l / (B \cdot Y),$$

wobei Y eine Materialkonstante ist, die von der Dehnungscharakteristik des Bandmaterials abhängig ist. Daraus lässt sich der ausgeübte Kompressionsdruck p in Abhängigkeit der relativen Dehnung $\varepsilon = s/l$ des Bands wie folgt beschreiben:

$$p = 2\pi\, F / (U \cdot B) = 2\pi\, s \cdot Y / (U \cdot l) = 2\pi \cdot \varepsilon \cdot Y / U,$$

Bei vorgegebenem Umfang U der Körperextremität an der Stelle des betreffenden Bands 3 ist der ausgeübte Kompressionsdruck p damit direkt proportional zur relativen Dehnung $\varepsilon = s/l$ des Bands und im Übrigen von dem Materialparameter Y des Kompressionstextils 1 und dem Umfang U der der Körperextremität an der Stelle des betreffenden Bands 3 abhängig. Der Umfang U der Körperextremität kann an jeder Stelle, an der sich ein Band 3 befindet gemessen werden, so dass bei bekanntem Materialparameter Y der bei einer bestimmten relativen Dehnung $\varepsilon$ erzeugte Kompressionsdruck p ermittelt werden kann.

Aus dieser Überlegung lässt sich die notwendige Zugkraft $F_{soll}$ errechnen, die auf die einzelnen Bänder 3 ausgeübt werden muss, um einen gewünschten Kompressionsdruck $p_{soll}$ an der Stelle des betreffenden Bands auf die Körperextremität zu erzeugen:

$$F_{soll} = p_{soll} \cdot (U \cdot B) / 2\pi.$$

Bei einem Solldruck von z.B. $p_{soll}$ = 40 mmHg und einer vorgegebenen Breite des Bands von B = 6,5 cm ergibt sich in Abhängigkeit des Umfangs U der Körperextremität eine notwendige Zugkraft $F_{soll}$ = 13 N.

Zweckmäßig können anhand einer Tabelle je nach Umfang der Körperextremität verschiedene Bereiche vordefiniert werden, wie aus dem Beispiel der folgenden Tabelle 1 ersichtlich, in der für verschiedene Umfangsbereiche die zugehörigen Dehnungsbereiche der absoluten und der relativen Dehnung angegeben sind, um bei einem bekannten Materialparameter Y (der bspw. bei Y = 15 N/cm liegt) einen einheitlichen, vorgegebenen Kompressionsdruck von bspw. p = 40 mm Hg zu erzielen:

**Tabelle 1:**

| Größe | 1/XS | | 2/S | | 3/M | | 4/L | | 5/XL | |
|---|---|---|---|---|---|---|---|---|---|---|
| Umfang U (cm) | 20,0 - 27,0 | | 25,0 - 32,0 | | 30,0 - 38,0 | | 37,0 - 44,0 | | 45,0 - 52,0 | |
| Dehnung s (cm) | 2,3 | 4,1 | 3,5 | 5,8 | 5,1 | 8,1 | 7,7 | 10,9 | 11,4 | 15,2 |
| Rel. Dehnung $\varepsilon$ | 11,3% | 15,2% | 14,1% | 18,0% | 16,9% | 21,4% | 20,8% | 24,8% | 25,3% | 29,3% |

Der Materialparameter Y kann dabei empirisch bzw. durch Messung der Dehnungscharakteristik (Dehnung s in Abhängigkeit der Zugkraft F) an Proben des Materials der Bänder 3 bzw. des Grundkörpers 2 ermittelt werden.

Bei einem Kompressionstextil mit einer Mehrzahl von Bändern 3a, 3b, 3c, 3d kann bei einem vorgegebenem maximalen Solldruck (von z.B. $p_{max}$ = 40 mmHg ) in Abhängigkeit des Verlaufs des Umfangs U(z) der Körperextremität entlang der Längsrichtung z (der bspw. mittels eines Maßbands leicht gemessen werden kann) die Breite $B(z_i)$ der einzelnen Bänder an der jeweiligen Position $z_i$ des Bands so bestimmt werden, dass sich bei Anwendung einer einheitlichen Zugkraft (von z.B. F = 13 N) auf das jeweilige Band ein Druckverlauf des vom Kompressionstextil auf die Körperextremität ausgeübten Kompressionsdrucks mit einem vordefinierten Druckprofil ergibt:

$$B(z_i) = 2\pi F / (U(z_i) \cdot p_{max}),$$

wobei $z_i$ feste Positionen entlang der Längsrichtung z der Körperextremität sind und die Lage der einzelnen Bänder (zweckmäßig die Mitte des jeweiligen Bands in Längsrichtung) definieren und $p_{max}$ der vordefinierte maximale Solldruck ist.

Ein Beispiel hierfür ist für einen maximalen Solldruck von $p_{max}$ = 40 mmHg = 0,53 N/cm$^2$, den das distale Band 3a auf die Körperextremität mit einem vorgegebenen Verlauf des Umfangs U(z) in Längsrichtung z ausüben soll, in der folgenden Tabelle 2 für einen Anwendungsfall an einem Unterschenkel angegeben, wobei der Verlauf des Kompressionsdrucks vom distalen Band 3a (welches 100% des distalen maximalen Solldrucks $p_{max}$ im Bereich des Knöchels ausübt) kontinuierlich bis zum proximalen Band 3d (das im Bereich unterhalb des Knies liegt) auf die Hälfte abnimmt (d.h. der vom proximalen Band 3d ausgeübte Druck beträgt 50% des maximalen distalen Solldrucks $p_{max}$, der vom distalen Band 3a ausgeübt wird).

**Tabelle 2:**

| Band | Position ($z_i$) | Breite (B)/cm | Solldruck ($p_{soll}$) (% von $p_{max}$ / N/cm$^2$) | Umfang (U)/ cm |
|---|---|---|---|---|
| 3d | cD (proximal) | 8,0 | 50 % / 0,27 | 38,0 |
| 3c | cC | 6,4 | 60 % / 0,32 | 40,0 |
| 3b | cB1 | 7,1 | 90 % / 0,48 | 24,0 |
| 3a | cB (distal) | 6,5 | 100% / 0,53 | 23,5 |

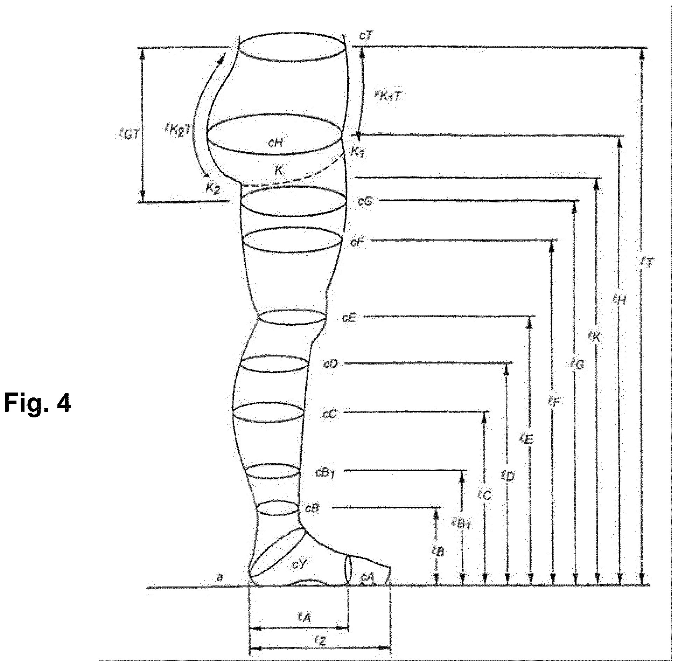

Die Lage der einzelnen Bänder 3a - 3d an der Körperextremität ist dabei zweckmäßig so ausgewählt, dass die (mittlere) Position $z_i$ der Bänder 3a - 3d zumindest annähernd mit den standardisierten Messpunkten (cB, cB1, cC, cD; gemäß RAL-GZ 387/1 ) zur Erfassung des Kompressionsdrucks bzw. des Druckprofils von medizinischen Kompressionsstrümpfen übereinstimmt (wie in der Spalte "Position ($z_i$)" in Tabelle 2 angegeben, wobei die Position "cB" die distale Position am Knöchel und die Position "cD" die proximale Position knapp unterhalb des Kniegelenks ist und die Positionen "cB1" und "cC" zwischen den Positionen "cB" und "cD" liegen, wie aus Figur 4 ersichtlich).

Die Erfindung ist nicht auf das hier zeichnerisch dargestellte Ausführungsbeispiel beschränkt. So kann bspw. die Anzahl der Bänder 3 des Kompressionstextils 1 variieren, wobei auch nur ein einziges Band 3 vorhanden sein kann. Die Ausdehnung des Kompressionstextils 1 entlang der Längsrichtung L der Körperextremität kann ebenfalls von dem zeichnerisch dargestellten Ausführungsbeispiel, in dem sich das Kompressionstextil an einem Unterschenkel zwischen dem Knöchel und dem Kniegelenk erstreckt, abweichen. So können bspw. je nach Anwendungsfall sowohl längere als auch kürzere Ausdehnungen des Kompressionstextils 1 entlang der Längsrichtung L der Körperextremität vorgesehen sein. Weiterhin können neben der in Längsrichtung L in einer Reihe nebeneinander angeordneten Bandreihe mit den Bändern 3a bis 3d noch eine oder mehrere weitere Bandreihen vorhanden sein, die entweder an demselben Endabschnitt (2a oder 2b) oder an jeweils gegenüberliegenden Endabschnitten (2a und 2b) angeordnet sein können. Weiterhin kann in Längsrichtung L zwischen benachbarten Bändern ein größerer Abstand vorgesehen sein wie in dem zeichnerisch dargestellten Ausführungsbeispiel. Ein kleiner Abstand, insbesondere ein schmaler Schlitz zwischen benachbarten Bändern, hat jedoch den Vorteil eines gleichmäßigen Kompressionsdrucks entlang der Längsrichtung L oder eines stetigen, graduellen Druckverlaufs.

Die angegebenen Materialen und Materialparameter sind nur beispielhaft zu verstehen und können an den jeweiligen Anwendungsfall angepasst werden.

An Stelle der in dem Ausführungsbeispiel verwendeten Klettverschlüsse als erste und zweite Befestigungsmittel können auch andere lösbare Befestigungsmittel, wie z.B. Hakenverschlüsse, Adhäsionsverschlüsse, Druckknöpfe oder Klammern verwendet werden.

Die zweiten Befestigungsmittel 5 können auch einstückig mit dem Verbindungselement 11 der Kraftmesseinrichtung 10 ausgeformt bzw. fest mit diesem verbunden sein.

Die Kraftmesseinrichtung 10 kann anstelle eines mechanischen Kraftaufnehmers 12 auch einen elektrischen Kraftaufnehmer enthalten, z.B. einen resistiven, induktiven, kapazitiven oder piezoelektrischen Kraftaufnehmer, wobei in diesem Fall bevorzugt eine (digitale) Anzeigeeinrichtung, insbesondere ein Display, zur Anzeige der aufgebrachten Zugkraft vorgesehen ist. Dabei ist es auch möglich, dass ein (insbesondere akustisches oder optisches) Signal abgegeben wird, sobald ein vordefinierter Wert der Zugkraft und/oder der Dehnung des Bands oder der vom gedehnten Band auf die Körperextremität ausgeübten Kompression erreicht oder überschritten ist.

**Patentansprüche**

1. Kompressionssystem zur Ausübung einer einstellbaren Kompression auf eine Körperextremität, umfassend ein zumindest bereichsweise elastisches Kompressionstextil (1) mit einem Grundkörper (2) und mindestens einem an dem Grundkörper angeordneten Band (3), welches über ein freies Ende verfügt, an dem erste Befestigungsmittel (4) angeordnet sind, wobei das Kompressionstextil (1) unter Dehnung um die Körperextremität schlingbar und unter Beibehaltung der Dehnung mittels der ersten Befestigungsmittel (4) an der Körperextremität festlegbar ist und sich das Kompressionstextil (1) beim Anlegen an die Körperextremität in Abhängigkeit einer zur Erzeugung der Dehnung (s) auf das Band (3) ausgeübten Zugkraft (F) dehnt, so dass bei angelegtem Kompressionstextil (1) eine von der Dehnung (s) abhängige Kompression auf die Körperextremität ausgeübt wird, **gekennzeichnet durch** eine Kraftmesseinrichtung (10) zur Aufnahme der beim Anlegen an die Körperextremität auf das Band (3) oder jedes Band (3a, 3b, 3c) ausgeübten Zugkraft (F), wobei die Kraftmesseinrichtung (10) mittels zweiter Befestigungsmittel (5) abnehmbar an dem freien Ende des mindestens einen Bands (3) oder jedes Bands (3a, 3b, 3c) befestigbar ist.

2. Kompressionssystem nach Anspruch 1, **dadurch gekennzeichnet, dass** an dem Grundkörper (2) eine Mehrzahl von Bändern (3a, 3b, 3c) angeordnet ist, wobei jedes der Bänder (3a, 3b, 3c) über ein freies Ende verfügt, an dem erste Befestigungsmittel (4) angeordnet sind, und das andere Ende jedes Bands (3a, 3b, 3c) mit dem Grundkörper (2) verbunden ist.

3. Kompressionssystem nach Anspruch 1 oder 2, **dadurch gekennzeichnet, dass** die Kraftmesseinrichtung (10) einen mechanischen Kraftaufnehmer (12), insbesondere ein Federkraftmesser, insbesondere einen Federkörper-Kraftaufnehmer enthält.

4. Kompressionssystem nach Anspruch 1 oder 2, **dadurch gekennzeichnet, dass** die Kraftmesseinrichtung (10) einen resistiven, induktiven, kapazitiven oder piezoelektrischen Kraftaufnehmer enthält.

5. Kompressionssystem nach Anspruch 2, **dadurch gekennzeichnet, dass** die Mehrzahl der Bänder (3) mindestens zwei, bevorzugt drei oder mehr Bänder (3a, 3b, 3c) umfasst, wobei sich die Bänder (3a, 3b, 3c) bei an der Körperextremität angelegtem Kompressionstextil (1) jeweils in Umfangsrichtung der Körperextremität erstrecken und in einer Längsrichtung (L) nebeneinander liegend angeordnet sind.

6. Kompressionssystem nach Anspruch 5, **dadurch gekennzeichnet, dass** in Längsrichtung (L) des Grundkörpers (2) benachbart nebeneinander liegende Bänder (3a, 3b, 3c) durch einen Schlitz im Grundkörper (2) des Kompressionstextils (1) voneinander getrennt sind.

7. Kompressionssystem nach einem der voranstehenden Ansprüche, **dadurch gekennzeichnet, dass** die ersten Befestigungsmittel (4) als Klettverschlüsse mit einem Hakenteil (4a) ausgebildet sind, wobei bevorzugt am freien Ende des Bands (3) oder jedes Bands (3a, 3b, 3c) ein Hakenteil (4a) befestigt ist.

8. Kompressionssystem nach einem der voranstehenden Ansprüche, **dadurch gekennzeichnet, dass** der Grundkörper (2) des Kompressionstextils (1) elastisch ist.

9. Kompressionssystem nach einem der voranstehenden Ansprüche, **dadurch gekennzeichnet, dass** das Band (3) oder die Bänder (3a, 3b, 3c) zumindest im Wesentlichen unelastisch sind oder eine geringere Elastizität aufweisen als der Grundkörper (2).

10. Kompressionssystem nach einem der voranstehenden Ansprüche, **dadurch gekennzeichnet, dass** die zweiten Befestigungsmittel (5) lösbar ausgebildet und insbesondere abnehmbar an den freien Enden des Bands (3) oder jedes der Bänder (3a, 3b, 3c) befestigt sind, insbesondere mittels Klettverschlüsse oder Druckknöpfe.

11. Kompressionssystem nach einem der voranstehenden Ansprüche, **dadurch gekennzeichnet, dass** die Kraftmesseinrichtung (10) ein Verbindungselement (11), insbesondere in Form einer Lasche, zur lösbaren Befestigung der Kraftmesseinrichtung (10) am freien Ende eines Bands (3; 3a, 3b oder 3c) aufweist, wobei die Zugkraft beim Anlegen des Kompressionstextils (1) an die Körperextremität von der Kraftmesseinrichtung (10) über das Verbindungselement (11) auf das mit dem Verbindungselement (11) verbundene Band (3; 3a, 3b oder 3c) übertragbar ist.

12. Kompressionssystem nach einem der voranstehenden Ansprüche, **dadurch gekennzeichnet, dass** die Kraftmesseinrichtung (10) einen Handgriff (13) oder eine Schlaufe aufweist.

13. Kompressionssystem nach einem der Ansprüche 11 oder 12, **dadurch gekennzeichnet, dass** das Verbindungselement (11) die zweiten Befestigungsmittel (5) zur lösbaren Befestigung des Verbindungselements (11) am freien Ende des oder jedes Bands (3; 3a, 3b oder 3c) umfasst.

## Claims

1. Compression system for applying adjustable compression to a body extremity, comprising an at least regionally elastic compression textile (1) with a base body (2) and at least one band (3) arranged on the base body and having a free end on which first fastening means (4) are arranged, wherein the compression textile (1) can be wrapped around the body extremity while being stretched and can be fixed to the body extremity while maintaining the stretch by means of the first fastening means (4), and the compression textile (1) stretches when applied to the body extremity as a function of a tensile force (F) exerted on the band (3) to generate the stretch (s), so that, when the compression textile (1) is applied, a compression dependent on the stretch (s) is exerted on the body extremity, **characterized by** a force measuring device (10) for recording the compression exerted on the band (3) or each band (3a, 3b, 3c) when applied to the body extremity, the force measuring device (10) being removably attachable to the free end of the at least one band (3) or each band (3a, 3b, 3c) by means of second attachment means (5).

2. Compression system according to claim 1, **characterized in that** a plurality of bands (3a, 3b, 3c) are arranged on said base body (2), each of said bands (3a, 3b, 3c) having a free end on which first fastening means (4) are arranged, and the other end of each band (3a, 3b, 3c) being connected to said base body (2).

3. Compression system according to claim 1 or 2, **characterized in that** the force measuring device (10) includes a mechanical force transducer (12), in particular a spring force transducer.

4. Compression system according to claim 1 or 2, **characterized in that** the force measuring device (10) includes a resistive, inductive, capacitive or piezoelectric force transducer.

**5.** Compression system according to claim 2, **characterized in that** the plurality of bandbands (3) comprises at least two, preferably three or more bandbands (3a, 3b, 3c), wherein the bands (3a, 3b, 3c) each extend in the circumferential direction of the body extremity when the compression textile (1) is applied to the body extremity and are arranged adjacent to each other in a longitudinal direction (L).

**6.** Compression system according to claim 5, **characterized in that** the bands (3a, 3b, 3c) lying adjacent to each other in the longitudinal direction (L) of the base body (2) are separated from each other by a slit in the base body (2) of the compression textile (1).

**7.** Compression system according to any one of the preceding claims, **characterized in that** the first fastening means (4) are formed as hook-and-loop fasteners with a hook part (4a), preferably a hook part (4a) being fastened to the free end of the band (3) or of each band (3a, 3b, 3c).

**8.** Compression system according to any one of the preceding claims, **characterized in that** the base body (2) of the compression textile (1) is elastic.

**9.** Compression system according to any one of the preceding claims, **characterized in that** the band (3) or the bands (3a, 3b, 3c) are at least substantially inelastic or have a lower elasticity than the base body (2).

**10.** Compression system according to any one of the preceding claims, **characterized in that** the second fastening means (5) are detachably formed and in particular removably fastened to the free ends of the band (3) or each of the bands (3a, 3b, 3c), in particular by means of hook-and-loop fasteners or snap fasteners.

**11.** Compression system according to any one of the preceding claims, **characterized in that** the force measuring device (10) has a connecting element (11), in particular in the form of a strap, for detachable attachment of the force measuring device (10) to the free end of a band (3; 3a, 3b or 3c), wherein the tensile force can be transmitted from the force measuring device (10) via the connecting element (11) to the band (3; 3a, 3b or 3c) connected to the connecting element (11) when the compression textile (1) is applied to the body extremity.

**12.** Compression system according to any one of the preceding claims, **characterized in that** the force measuring device (10) comprises a handle (13) or a loop.

**13.** A compression system according to any one of claims 11 or 12, **characterised in that** the connecting element (11) comprises the second fastening means (5) for releasably fastening the connecting element (11) to the free end of the or each band (3; 3a, 3b or 3c).

**Revendications**

**1.** Système de compression destiné à exercer une compression réglable sur une extrémité de corps, comprenant un textile de compression (1) élastique au moins par endroits avec un corps de base (2) et au moins une bande (3) agencée contre le corps de base, laquelle dispose d'une extrémité libre contre laquelle sont agencés des premiers moyens de fixation (4), dans lequel le textile de compression (1) peut être enroulé avec étirement autour de l'extrémité de corps et peut être fixé tout en maintenant l'étirement contre l'extrémité de corps au moyen des premiers moyens de fixation (4) et le textile de compression (1) s'étire, lors de l'application contre l'extrémité de corps, en fonction d'une force de traction (F) exercée pour la production de l'étirement (s) sur la bande (3), de sorte que, lorsque le textile de compression (1) est appliqué, une compression dépendante de l'étirement (s) soit exercée sur l'extrémité de corps, **caractérisé par** un équipement dynamométrique (10) pour l'enregistrement de la force de traction (F) exercée, lors de l'application contre l'extrémité de corps, sur la bande (3) ou chaque bande (3a, 3b, 3c), dans lequel l'équipement dynamométrique (10) peut être fixé au moyen de seconds moyens de fixation (5) pouvant être retirés au niveau de l'extrémité libre de la au moins une bande (3) ou de chaque bande (3a, 3b, 3c).

**2.** Système de compression selon la revendication 1, **caractérisé en ce qu'**une pluralité de bandes (3a, 3b, 3c) est agencée contre le corps de base (2), dans lequel chacune des bandes (3a, 3b, 3c) dispose d'une extrémité libre contre laquelle sont agencés des premiers moyens de fixation (4), et l'autre extrémité de chaque bande (3a, 3b, 3c) est reliée au corps de base (2).

**3.** Système de compression selon la revendication 1 ou 2, **caractérisé en ce que** l'équipement dynamométrique (10) contient un capteur de force (12) mécanique, en particulier un dynamomètre à ressort, en particulier un capteur de force avec corps à ressort.

4. Système de compression selon la revendication 1 ou 2, **caractérisé en ce que** l'équipement dynamométrique (10) contient un capteur de force résistif, inductif, capacitif ou piézoélectrique.

5. Système de compression selon la revendication 2, **caractérisé en ce que** la pluralité des bandes (3) comprend au moins deux, de préférence trois ou davantage de bandes (3a, 3b, 3c), dans lequel les bandes (3a, 3b, 3c), lorsque le textile de compression (1) est appliqué contre l'extrémité de corps, s'étendent respectivement dans la direction circonférentielle de l'extrémité de corps et sont agencées côte à côte dans une direction longitudinale (L).

6. Système de compression selon la revendication 5, **caractérisé en ce que** dans la direction longitudinale (L) du corps de base (2), des bandes (3a, 3b, 3c) côte à côte de façon avoisinante sont séparées par une encoche dans le corps de base (2) du textile de compression (1).

7. Système de compression selon l'une des revendications précédentes, **caractérisé en ce que** les premiers moyens de fixation (4) sont conçus en tant que fermetures auto-agrippantes avec une partie formant crochet (4a), dans lequel une partie formant crochet (4a) est fixée de préférence au niveau de l'extrémité libre de la bande (3) ou de chaque bande (3a, 3b, 3c) .

8. Système de compression selon l'une des revendications précédentes, **caractérisé en ce que** le corps de base (2) du textile de compression (1) est élastique.

9. Système de compression selon l'une des revendications précédentes, **caractérisé en ce que** la bande (3) ou les bandes (3a, 3b, 3c) sont au moins essentiellement non élastiques ou présentent une élasticité inférieure à celle du corps de base (2).

10. Système de compression selon l'une des revendications précédentes, **caractérisé en ce que** les seconds moyens de fixation (5) sont conçus de façon amovible et en particulier fixés de façon à pouvoir être retirés au niveau des extrémités libres de la bande (3) ou de chacune des bandes (3a, 3b, 3c), en particulier au moyen de fermetures auto-agrippantes ou de boutons-poussoirs.

11. Système de compression selon l'une des revendications précédentes, **caractérisé en ce que** l'équipement dynamométrique (10) présente un élément de liaison (11), en particulier sous la forme d'une patte, pour la fixation amovible de l'équipement dynamométrique (10) au niveau de l'extrémité libre d'une bande (3; 3a, 3b ou 3c), dans lequel la force de traction, lorsque le textile de compression (1) est appliqué contre l'extrémité de corps, peut être transmise depuis l'équipement dynamométrique (10) via l'élément de liaison (11) à la bande (3; 3a, 3b ou 3c) reliée à l'élément de liaison (11).

12. Système de compression selon l'une des revendications précédentes, **caractérisé en ce que** l'équipement dynamométrique (10) présente une poignée (13) ou une boucle.

13. Système de compression selon l'une des revendications 11 ou 12, **caractérisé en ce que** l'élément de liaison (11) comprend les seconds moyens de fixation (5) pour la fixation amovible de l'élément de liaison (11) au niveau de l'extrémité libre de la ou de chaque bande (3; 3a, 3b ou 3c).

**Fig. 1**

Fig. 2

Fig. 3b

Fig. 3a

**Fig. 3c**

**Fig. 3d**

EP 3 858 307 B1

Fig. 3e

Fig. 3f

Fig. 3g

Fig. 3h

Fig. 4

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 6338723 B1

- DE 102009016513 A1

**In der Beschreibung aufgeführte Nicht-Patentliteratur**

- **E. RABE.** Medizinische Kompressionstherapie der Extremitäten mit Medizinischem Kompressions-strumpf (MKS), Phlebologischem Kompressionsver-band (PKV) und Medizinischen adaptiven Kompres-sionssystemen (MAK). *Deutschen Gesellschaft für Phlebologie e.V. (DGP), www.awmf.org/leitlinien/detail/ll/037-005.html*